# DEMANDE DE BREVET EUROPEEN

(11) **EP 3 922 709 A1**
(43) Date de publication de la demande: **15.12.2021**
(21) Numéro de dépôt: 21178911.0
(22) Date de dépôt: 11.06.2021
(51) Int. Cl.: C12M 1/32, C12M 1/00, C12M 1/34

(54) **BOÎTE DE CULTURE**

(30) Priorité: 12.06.2020 FR 2006151
(71) Demandeur: Commissariat à l'Energie Atomique et aux Energies Alternatives, 75015 Paris (FR); Institut Polytechnique de Grenoble, 38000 Grenoble (FR); Centre national de la recherche scientifique, 75016 Paris (FR)
(72) Inventeur: MAINDRON, Tony, 38054 GRENOBLE CEDEX 09 (FR); BRUCKERT, Franz, 38016 GRENOBLE CEDEX 1 (FR); WEIDENHAUPT, Marianne, 38016 GRENOBLE CEDEX 1 (FR)
(74) Mandataire: Cabinet Beaumont

(57) **Abrégé**

La présente description concerne une boîte de culture (100) comportant : une matrice de sources lumineuses (112) formée dans et sur un substrat semiconducteur ; une couche transparente de planarisation revêtant la matrice de sources lumineuses ; et une matrice de puits (102) formée sur ladite couche, chaque source lumineuse étant située à l'aplomb d'un puits.

## Description

### Domaine technique

La présente description concerne de façon générale les dispositifs électroniques et, plus particulièrement, les dispositifs de stimulation de cellules par la lumière.

### Technique antérieure

Dans des domaines tels que l'optogénétique, on utilise souvent des sources lumineuses associées à un microscope afin d'illuminer des cellules en culture. Des tissus d'organismes vivants peuvent alternativement être illuminés grâce à des fibres optiques reliées à une source lumineuse. On connaît en outre des outils couplés à la microscopie qui permettent un adressage spatial de la lumière émise. Les dispositifs d'illumination actuels demeurent toutefois limités, notamment en termes de surface illuminée et de paramètres d'illumination pouvant être testés en parallèle.

### Résumé de l'invention

Il existe un besoin d'améliorer les dispositifs d'illumination de cellules et leurs procédés de fabrication actuels.

Un mode de réalisation pallie tout ou partie des inconvénients des dispositifs d'illumination de cellules et de leurs procédés de fabrication connus.

Un mode de réalisation prévoit une boîte de culture comportant :
- une matrice de sources lumineuses formée dans et sur un substrat semiconducteur ;
- une couche transparente de planarisation revêtant la matrice de sources lumineuses ; et
- une matrice de puits formée sur ladite couche, chaque source lumineuse étant située à l'aplomb d'un puits.

Selon un mode de réalisation, ladite couche est en un matériau organosilicié photoréticulable.

Selon un mode de réalisation, ladite couche présente une épaisseur comprise entre 1 µm et 10 µm.

Selon un mode de réalisation, la matrice de puits est en polydiméthylsiloxane.

Selon un mode de réalisation, chaque source lumineuse comporte au moins une diode électroluminescente organique.

Selon un mode de réalisation, chaque source lumineuse comporte une diode destinée à émettre une lumière blanche.

Selon un mode de réalisation, chaque diode est commandable individuellement.

Selon un mode de réalisation, chaque source lumineuse comporte trois diodes destinées à émettre respectivement une lumière de couleur rouge, une lumière de couleur verte et une lumière de couleur bleue.

Selon un mode de réalisation, la matrice de sources lumineuses est située du côté d'une première face du substrat, opposée à une deuxième face du substrat comportant des plots de connexion.

Selon un mode de réalisation, les plots de connexion sont connectés à la première face du substrat par des vias traversants.

Selon un mode de réalisation, chaque source lumineuse comporte un circuit de commande indépendant des autres sources lumineuses.

Selon un mode de réalisation, le fond et/ou les parois latérales d'au moins une partie des puits de la matrice sont fonctionnalisés.

Un mode de réalisation prévoit un procédé de fabrication d'une boîte telle que décrite.

Selon un mode de réalisation, le procédé comporte les étapes suivantes :
- former la matrice de sources lumineuses dans et sur le substrat semiconducteur ;
- revêtir la matrice de sources lumineuses de la couche transparente de planarisation ; et
- former en surface de ladite couche un puits à l'aplomb de chaque source lumineuse.

### Brève description des dessins

Ces caractéristiques et avantages, ainsi que d'autres, seront exposés en détail dans la description suivante de modes de réalisation et modes de mise en œuvre particuliers faite à titre non limitatif en relation avec les figures jointes parmi lesquelles :
la figure 1 est une vue en perspective, schématique et partielle, d'un mode de réalisation d'une boîte de culture multipuits ;
la figure 2 est une vue en coupe, schématique et partielle, illustrant une étape d'un mode de mise en œuvre d'un procédé de fabrication de la boîte de culture de la figure 1 ;
la figure 3 est une vue en coupe, schématique et partielle, illustrant une autre étape du mode de mise en œuvre du procédé de fabrication de la boîte de culture de la figure 1 ;
la figure 4 est une vue en coupe, schématique et partielle, illustrant encore une autre étape du mode de mise en œuvre du procédé de fabrication de la boîte de culture de la figure 1 ;
la figure 5 est une vue en coupe, schématique et partielle, illustrant encore une autre étape du mode de mise en œuvre du procédé de fabrication de la boîte de culture de la figure 1 ;
la figure 6 est une vue en coupe, schématique et partielle, illustrant encore une autre étape du mode de mise en œuvre du procédé de fabrication de la boîte de culture de la figure 1 ; et
la figure 7 est une vue en coupe, schématique et partielle, illustrant encore une autre étape du mode de mise en œuvre du procédé de fabrication de la boîte de culture de la figure 1.

### Description des modes de réalisation

De mêmes éléments ont été désignés par de mêmes références dans les différentes figures. En particulier, les éléments structurels et/ou fonctionnels communs aux différents modes de réalisation et modes de mise en œuvre peuvent présenter les mêmes références et peuvent disposer de propriétés structurelles, dimensionnelles et matérielles identiques.

Par souci de clarté, seuls les étapes et éléments utiles à la compréhension des modes de réalisation et modes de mise en œuvre décrits ont été représentés et sont détaillés. En particulier, les diodes électroluminescentes organiques et leurs procédés de fabrication ne sont pas détaillés, les modes de réalisation et modes de mise en œuvre étant compatibles avec les diodes électroluminescentes organiques et leurs procédés de fabrication usuels.

Sauf précision contraire, lorsque l'on fait référence à deux éléments connectés entre eux, cela signifie directement connectés sans éléments intermédiaires autres que des conducteurs, et lorsque l'on fait référence à deux éléments reliés (en anglais "coupled") entre eux, cela signifie que ces deux éléments peuvent être connectés ou être reliés par l'intermédiaire d'un ou plusieurs autres éléments.

Dans la description qui suit, lorsque l'on fait référence à des qualificatifs de position absolue, tels que les termes "avant", "arrière", "haut", "bas", "gauche", "droite", etc., ou relative, tels que les termes "dessus", "dessous", "supérieur", "inférieur", etc., ou à des qualificatifs d'orientation, tels que les termes "horizontal", "vertical", etc., il est fait référence sauf précision contraire à l'orientation des figures ou à une boîte de culture dans une position normale d'utilisation.

Sauf précision contraire, les expressions "environ", "approximativement", "sensiblement", et "de l'ordre de" signifient à 10 % près, de préférence à 5 % près.

On désigne par facteur de transmission (ou « transmittance ») d'une couche le rapport entre l'intensité du rayonnement sortant de la couche et l'intensité du rayonnement entrant dans la couche. Dans la suite de la description, une couche est dite opaque à un rayonnement lorsque le facteur de transmission du rayonnement au travers de la couche est inférieur à 10 %. Dans la suite de la description, une couche est dite transparente à un rayonnement lorsque le facteur de transmission du rayonnement au travers de la couche est supérieur à 10 %.

On désigne par facteur de réflexion (ou « réflectance ») d'une couche le rapport entre le flux lumineux réfléchi par la couche et le flux lumineux incident sur la couche. Dans la suite de la description, une couche est dite réfléchissante lorsque le facteur de réflexion de la couche est supérieur à 90 %.

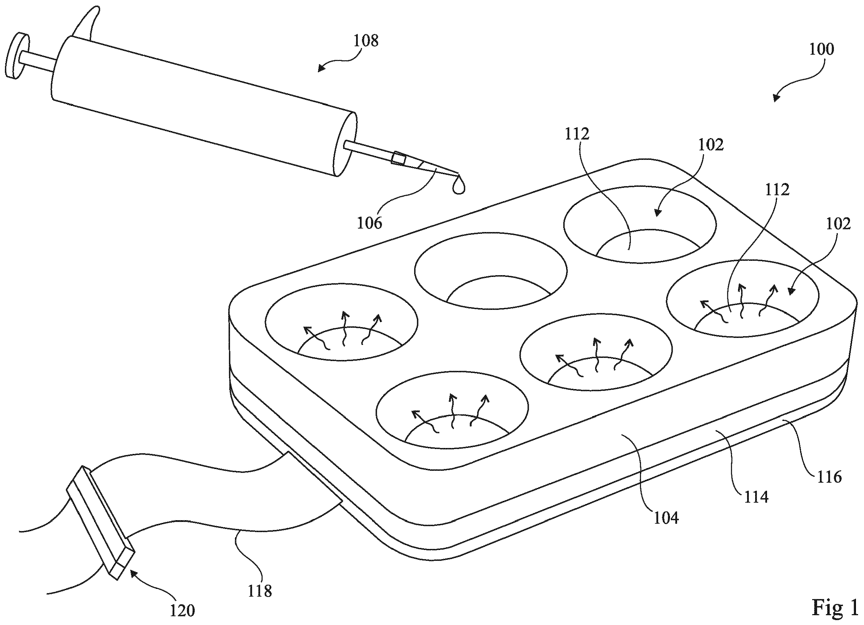

La figure 1 est une vue en perspective, schématique et partielle, d'un mode de réalisation d'une plaque ou boîte 100 de culture multipuits.

La boîte de culture 100 comporte une matrice de puits 102. Dans l'exemple représenté, chaque puits 102 présente une forme cylindrique (de section circulaire, en vue de dessus) s'étendant verticalement depuis la face supérieure de la boîte 100. Les puits 102 sont par exemple répartis de façon régulière sur la face supérieure de la boîte 100. À titre d'exemple, tous les puits 102 de la boîte 100 présentent des dimensions identiques, aux dispersions de fabrication près.

Dans l'exemple représenté, les puits 102 de la boîte 100 sont des évidements ou compartiments formés à l'intérieur d'une couche 104. Les puits 102 sont par exemple destinés à contenir une substance (ou milieu) biologique 106, comprenant par exemple des cellules. À titre d'exemple, la substance biologique 106 est dispensée à l'intérieur des puits 102 de la boîte 100 à l'aide d'une pipette 108 équipée d'un cône de pipetage adapté, par exemple, aux dimensions des puits 102.

La couche 104 est de préférence en un matériau biocompatible, autrement dit en un matériau non toxique pour les cellules contenues dans la substance biologique 106. À titre d'exemple, la couche 104 est en polydiméthylsiloxane (PDMS) ou en un polymère plastique, par exemple en polystyrène, en polypropylène, en polycarbonate, etc. La couche 104 peut être revêtue d'une fonctionnalisation chimique destinée à favoriser une adhésion des cellules contenues dans la substance 106. Par exemple, le fond et/ou les parois latérales d'au moins une partie des puits 102 sont fonctionnalisés.

La boîte de culture 100 comporte en outre une matrice de sources lumineuses 112. Dans l'exemple représenté, la matrice de sources lumineuses 112 est alignée verticalement par rapport à la matrice de puits 102. Chaque source lumineuse 112 est plus précisément située à l'aplomb d'un puits 102 auquel elle est associée. À titre d'exemple, le fond de chaque puits 102 est centré par rapport à la face supérieure de la source lumineuse 112 associée.

Dans l'exemple représenté, la matrice de sources lumineuses 112 est enrobée sur toutes ses faces latérales par une couche 114. À titre d'exemple, la couche 114 permet de protéger contre l'oxydation des éléments de reprise de contact (non représentés) associés aux sources lumineuses 112. La couche 114 est par exemple une couche dite de remplissage (underfill) en un matériau biocompatible. À titre d'exemple, la couche 114 est en une résine ou colle époxy, par exemple la colle connue sous la dénomination commerciale « Epo-Tek MED-375 ».

Les sources lumineuses 112 de la boîte 100 sont par exemple alimentées et commandées par l'intermédiaire d'une carte 116 de circuit imprimé. Dans l'exemple représenté, la carte 116 est située du côté de la face inférieure de la boîte 100, c'est-à-dire la face opposée à la matrice de puits 102. La carte 116 est par exemple reliée ou connectée à une unité de commande (non représentée en figure 1), par exemple par l'intermédiaire d'un câble en nappe 118 et d'un connecteur 120. À titre d'exemple, l'unité de commande est un ordinateur configuré pour fournir l'alimentation électrique et commander des paramètres d'éclairage des sources lumineuses 112 de la boîte de culture 100.

Selon un mode de réalisation, les sources lumineuses 112 de la boîte de culture 100 sont individuellement adressables. Chaque source lumineuse 112 peut en particulier émettre ou non de la lumière indépendamment des autres sources lumineuses 112 de la boîte 100. À titre d'exemple, chaque source lumineuse 112 comporte un circuit de commande (non représenté) qui lui est propre, c'est-à-dire un circuit de commande indépendant des circuits de commande des autres sources lumineuses 112 de la boîte 100.

En figure 1, on a symbolisé l'émission de lumière provenant d'une source lumineuse 112 par des flèches partant de la face supérieure de la source lumineuse 112 considérée et orientées en direction de l'orifice d'entrée du puits 102 associé. Dans l'exemple représenté, on suppose arbitrairement que quatre sources lumineuses 112 émettent de la lumière tandis que deux sources lumineuses 112 n'émettent pas de lumière.

Les paramètres d'éclairage de chaque source lumineuse 112 sont de préférence ajustables indépendamment des paramètres d'éclairage des autres sources lumineuses 112 de la boîte 100. À titre d'exemple, il est notamment possible de commander individuellement, c'est-à-dire pour chaque source lumineuse 112 de la boîte 100, des paramètres choisis parmi :
- une durée d'éclairage ;
- une longueur d'onde ou une plage de longueurs d'onde d'émission, autrement dit une couleur émise ;
- un niveau de luminance ou puissance d'émission ; et
- un mode d'émission, par exemple de façon continue ou en lumière pulsée, les modes d'émission de façon continue et en lumière pulsée pouvant par exemple être alternés lors d'une même phase d'exposition d'un puits 102.

De manière générale, la boîte de culture 100 permet de commander les conditions d'illumination à l'intérieur de chaque puits 102. On peut en particulier choisir d'illuminer ou non la substance biologique 106 contenue dans chaque puits 102 de la boîte 100 et commander individuellement les paramètres d'émission de chaque source lumineuse 112 en fonctionnement.

Un avantage de la boîte de culture 100 réside dans le fait qu'il est possible de faire varier, d'un puits 102 à l'autre, les conditions d'illumination auxquelles est soumise la substance biologique 106. Dans des applications telles que les études de réponse cellulaire à la lumière, l'optogénétique, la neuromodulation, la neuroprotection, le diagnostic et le séquençage, l'utilisation de la boîte 100 permet par exemple de simplifier avantageusement la mise en œuvre de plans d'expérience, en particulier par rapport à l'utilisation de plaques ou boîtes de culture dont tous les puits présenteraient des conditions d'illumination semblables.

Un autre avantage de la boîte de culture 100 tient au fait que les matériaux entrant dans la composition de ses faces externes sont par exemple compatibles avec des procédés de stérilisation par lumière ultraviolette ou par un gaz, par exemple l'oxyde d'éthylène. À titre d'exemple, cela permet de réutiliser une même boîte 100 pour effectuer des expériences mettant en œuvre des substances biologiques différentes.

Bien que l'on ait illustré en figure 1 un mode de réalisation dans lequel la boîte de culture 100 comporte six puits 102, la boîte 100 peut en pratique comporter un nombre quelconque de puits 102, par exemple quatre, huit, douze, vingt-quatre, quarante-huit ou quatre-vingt-seize puits 102. En outre, bien que l'on ait représenté en figure 1 une boîte de culture 100 comportant des puits 102 de forme cylindrique, la boîte 100 peut en pratique comporter des puits 102 de forme quelconque, par exemple des puits 102 présentant, vus de dessus, une section de forme rectangulaire ou carrée.

À titre d'exemple, chaque puits 102 présente :
- une dimension maximale (en vue de dessus) de l'ordre de quelques micromètres à quelques millimètres, par exemple comprise entre 5 mm et 40 mm ; et
- une profondeur comprise entre 100 µm et 30 mm, par exemple comprise entre 10 mm et 20 mm.

Chaque puits 102 présente par exemple :
- une surface (en vue de dessus) comprise entre 0,19 cm² et 13 cm², par exemple égale à environ 0,3 cm² pour une boîte 100 comportant quatre-vingt-seize puits 102 ; et
- un volume compris entre quelques microlitres et quelques millilitres, par exemple compris entre 0,1 mL et 20 mL, par exemple égal à environ 0,3 mL pour une boîte 100 comportant quatre-vingt-seize puits 102.

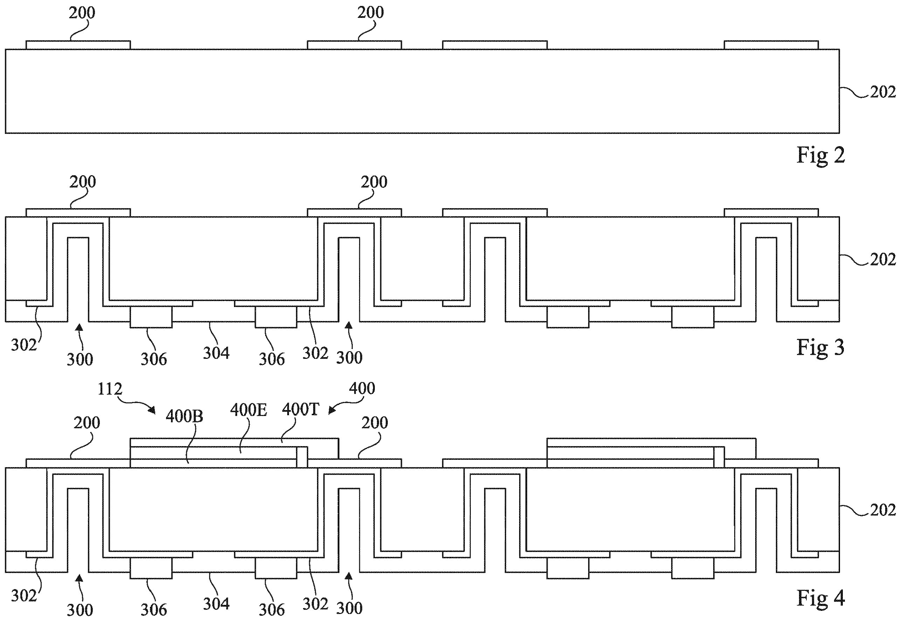

Les figures 2 à 7 ci-dessous illustrent des structures obtenues à l'issue d'étapes successives d'un exemple de mode de mise en œuvre d'un procédé de fabrication de la boîte de culture multipuits 100 de la figure 1.

La figure 2 est une vue en coupe, schématique et partielle, illustrant une étape du mode de mise en œuvre du procédé de fabrication de la boîte de culture 100 de la figure 1.

Au cours de cette étape, des circuits d'alimentation et d'adressage 200 sont réalisés dans et sur un substrat semiconducteur 202.

À titre d'exemple, le substrat 202 est une plaquette ou un morceau de plaquette en un matériau semiconducteur, par exemple en silicium. Dans l'orientation de la figure 2, les circuits 200 d'alimentation et d'adressage sont réalisés du côté de la face supérieure, ou face avant, du substrat 202. Ces circuits sont notamment destinés à permettre de commander individuellement chaque source lumineuse 112 (figure 1) de la boîte 100. À titre d'exemple, chaque source lumineuse 112 est associée à un circuit de commande 200 distinct des circuits de commande 200 des autres sources lumineuses 112 de la boîte 100.

La figure 3 est une vue en coupe, schématique et partielle, illustrant une autre étape du mode de mise en œuvre du procédé de fabrication de la boîte de culture 100 de la figure 1, réalisée à partir de la structure telle que décrite en relation avec la figure 2.

Au cours de cette étape, le substrat 202 est aminci et des vias 300 sont réalisés.

À titre d'exemple, l'amincissement du substrat 202 est effectué par meulage du côté de la face du substrat 202 ne comportant pas les circuits 200 d'alimentation et d'adressage (autrement dit la face inférieure du substrat 202, dans l'orientation de la figure 3). À l'issue de cette opération, le substrat 202 présente par exemple une épaisseur comprise entre 50 µm et 150 µm, de préférence entre 70 µm et 100 µm.

Dans l'exemple représenté, les vias 300 traversent le substrat 202 sur toute son épaisseur. Les vias 300 permettent par exemple de reporter en face inférieure du substrat 202 des élements de reprise de contact des circuits 200 situés du côté de la face supérieure du substrat 202.

Les parois latérales et le fond des vias traversants 300 sont par exemple revêtus de portions 302 d'une couche électriquement conductrice, par exemple une couche de cuivre. Dans l'exemple représenté, chaque portion 302 de couche conductrice se prolonge horizontalement sur et en contact avec la face inférieure du substrat 202, au voisinage du via 300 correspondant.

En pratique, la couche conductrice est par exemple déposée sur toute la structure du côté de la face inférieure du substrat 202. À titre d'exemple, les portions 302 de couche conductrice sont ensuite formées par gravure.

Le substrat 202 comporte en outre, du côté de sa face inférieure, une couche 304 électriquement isolante. La couche 304 recouvre des zones libres de la face inférieure du substrat 202, et revêt les portions 302 de couche conductrice à l'exception de régions sur et en contact avec lesquelles sont formés des plots 306 de connexion.

En pratique, la couche isolante 304 est par exemple d'abord déposée sur toute la structure du côté de la face inférieure du substrat 202. À titre d'exemple, des ouvertures sont ensuite pratiquées dans la couche 304 afin d'exposer les régions destinées à recevoir les plots 306 de connexion. Les plots 306 sont enfin réalisés à l'aplomb des régions non revêtues par la couche isolante 304.

Bien que cela ne soit pas représenté, une autre couche isolante peut être formée sur la structure, du côté de la face inférieure du substrat 202, préalablement au dépôt de la couche conductrice. Dans ce cas, cette couche est par exemple destinée à assurer une fonction d'isolation électrique entre les portions 302 de couche conductrice et le substrat 202.

La figure 4 est une vue en coupe, schématique et partielle, illustrant encore une autre étape du mode de mise en œuvre du procédé de fabrication de la boîte de culture 100 de la figure 1, réalisée à partir de la structure telle que décrite en relation avec la figure 3.

Au cours de cette étape, la matrice de sources lumineuses 112 est réalisée du côté de la face supérieure du substrat 202.

Selon un mode de réalisation, chaque source lumineuse 112 comporte au moins une diode électroluminescente organique 400, ou OLED (de l'anglais Organic Light-Emitting Diode). Dans l'exemple représenté, chaque source lumineuse 112 comporte une seule diode 400. Chaque diode 400 est par exemple destinée à émettre de la lumière blanche.

Pour simplifier, chaque diode 400 est symbolisée en figure 4 par un empilement comprenant une couche émissive 400E intercalée entre une électrode inférieure 400B et une électrode supérieure 400T. Dans l'exemple représenté, l'électrode inférieure 400B de chaque diode 400 est sur et en contact avec la face supérieure du substrat 202.

En pratique, les diodes 400 peuvent présenter une structure plus complexe. À titre d'exemple, chaque diode 400 peut en particulier comporter une ou plusieurs couches additionnelles (non représentées) telles que :
- une couche d'injection d'électrons (Electron Injection Layer - EIL) ;
- une couche de transport d'électrons (Electron Transport Layer - ETL) ;
- une couche d'injection de trous (Hole Injection Layer - HIL) ; et
- une couche de transport de trous (Hole Transport Layer - HTL).

Dans l'exemple représenté, les électrodes inférieure 400B et supérieure 400T de chaque diode électroluminescente organique 400 contactent respectivement des parties différentes des circuits 200 d'alimentation et de commande. Cela permet par exemple d'appliquer des potentiels électriques distincts sur l'électrode inférieure 400B et sur l'électrode supérieure 400T. Dans l'exemple représenté, les électrodes inférieure 400B et supérieure 400T de chaque diode 400 sont connectées à des plots 306 distincts par l'intermédiaire de vias 300 différents.

À titre d'exemple, l'électrode inférieure 400B de chaque diode 400 réfléchit la lumière émise par la couche 400E tandis que l'électrode supérieure 400T est transparente ou semi-transparente. La couche 400E présente par exemple une transmittance supérieure à 10 %. Dans l'exemple représenté, les diodes électroluminescentes organiques 400 sont dites à émission vers le haut (top emission, en anglais).

On a décrit en relation avec la figure 4 un mode de mise en œuvre dans lequel chaque source lumineuse 112 comporte une seule diode 400 émettant une lumière blanche. À titre de variante, chaque source lumineuse 112 comporte plusieurs diodes électroluminescentes organiques 400, par exemple trois diodes électroluminescentes organiques 400 destinées à émettre respectivement :
- une lumière de couleur rouge, autrement dit un rayonnement électromagnétique dont la longueur d'onde est par exemple comprise entre 610 nm et 700 nm ;
- une lumière de couleur verte, autrement dit un rayonnement électromagnétique dont la longueur d'onde est par exemple comprise entre 500 nm et 570 nm ; et
- une lumière de couleur bleue, autrement dit un rayonnement électromagnétique dont la longueur d'onde est par exemple comprise entre 450 nm et 490 nm.

Dans le cas où chaque source lumineuse 112 comporte plusieurs diodes électroluminescentes 400, chaque diode 400 est de préférence commandable individuellement. À titre d'exemple, chaque diode 400 appartenant à une même source lumineuse 112 est commandable indépendamment des autres diodes 400 de la source lumineuse 112 considérée, et indépendamment des diodes 400 faisant partie des autres sources lumineuses 112 de la boîte 100.

Un avantage lié à l'utilisation de diodes 400 organiques tient au fait que cela permet d'obtenir une faible dispersion spectrale d'émission, par exemple de l'ordre de 10 nm à 100 nm. Cette dispersion résulte par exemple de la structure des diodes organiques 400. On peut ainsi exciter la substance biologique 106 dans des plages de longueurs d'onde restreintes.

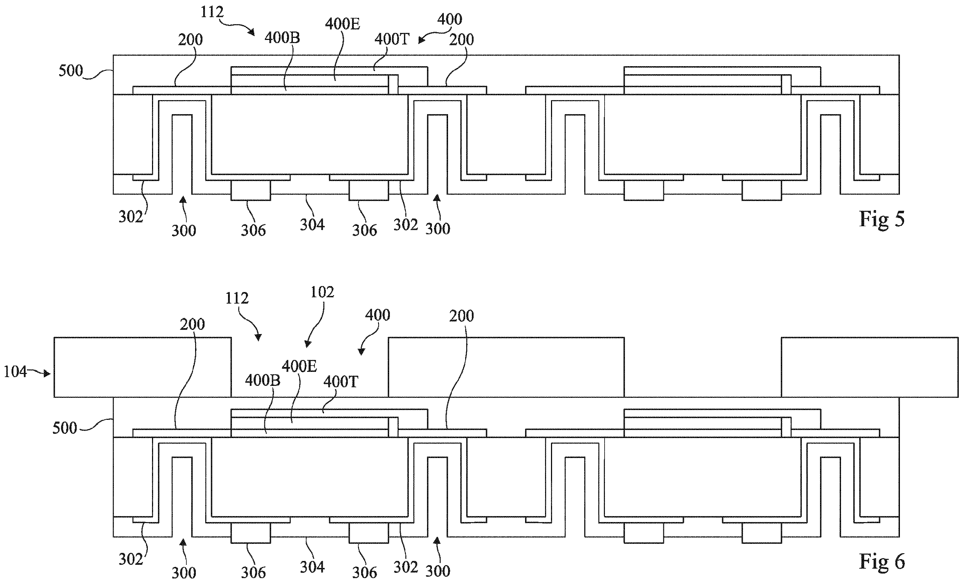

La figure 5 est une vue en coupe, schématique et partielle, illustrant encore une autre étape du mode de mise en œuvre du procédé de fabrication de la boîte de culture 100 de la figure 1, réalisée à partir de la structure telle que décrite en relation avec la figure 4.

Au cours de cette étape, la face supérieure de la structure est revêtue d'une couche 500 transparente de planarisation.

Dans l'exemple représenté, la couche 500 transparente de planarisation recouvre en particulier totalement les circuits 200 d'alimentation et de commande ainsi que les diodes électroluminescentes organiques 400. La couche 500 forme un revêtement dur (hard coat, en anglais).

Selon un mode de réalisation, la couche 500 est en un matériau organosilicié photoréticulable. À titre d'exemple, la couche 500 est à base d'un matériau connu sous la dénomination commerciale HARDRISE de la société POLYRISE ou une résine biocompatible. De manière plus générale, la couche 500 est en un matériau biocompatible. En pratique, la couche 500 est par exemple obtenue par la mise en œuvre d'un procédé de type sol-gel. La couche 500 présente par exemple une épaisseur comprise entre 1 µm et 10 µm.

Bien que cela ne soit pas représenté, une couche d'encapsulation des diodes électroluminescentes organiques 400 peut être déposée du côté de la face supérieure du substrat 202 préalablement au dépôt de la couche 500.

La figure 6 est une vue en coupe, schématique et partielle, illustrant encore une autre étape du mode de mise en œuvre du procédé de fabrication de la boîte de culture 100 de la figure 1, réalisée à partir de la structure telle que décrite en relation avec la figure 5.

Au cours de cette étape, la matrice de puits 102 est réalisée.

En pratique, la couche 104 est par exemple déposée sur toute la face supérieure de la structure. Les puits 102 sont ensuite formés de sorte à exposer des portions de couche 500 de planarisation situées à l'aplomb de chaque source lumineuse 112. Dans l'exemple représenté, le fond de chaque puits 102 est constitué par une portion de face supérieure de la couche 500.

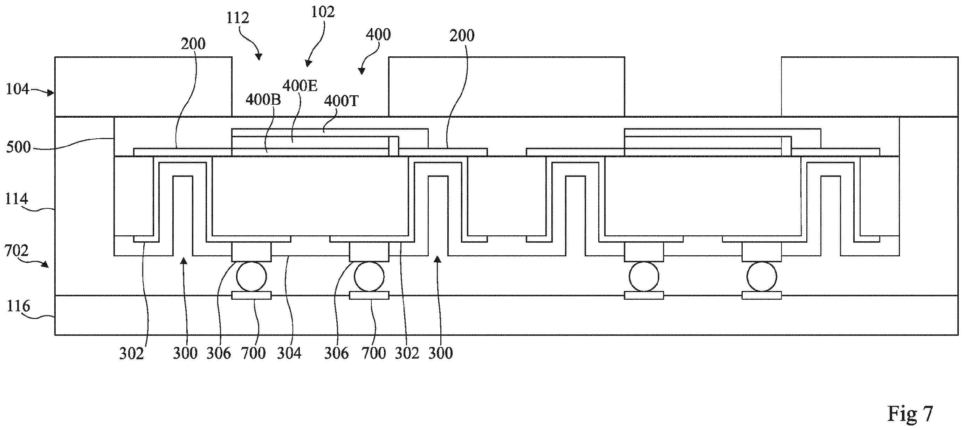

La figure 7 est une vue en coupe, schématique et partielle, illustrant encore une autre étape du mode de mise en œuvre du procédé de fabrication de la boîte de culture 100 de la figure 1, réalisée à partir de la structure telle que décrite en relation avec la figure 6.

Au cours de cette étape, la structure est liée à la carte de circuit imprimé 116 et la couche 114 de remplissage est formée.

Chaque plot 306 situé du côté de la face inférieure du substrat 202 est par exemple connecté à un plot 700 situé du côté de la face supérieure de la carte 116. À titre d'exemple, une matrice de billes de soudure 702 permet de réaliser la connexion entre les plots 306 et les plots 700.

Dans l'exemple représenté, la couche 114 revêt les faces latérales de la couche 500, du substrat 202 et de la couche 304. La couche 114 remplit en outre le volume libre délimité verticalement par la face supérieure de la carte 116 et par la face inférieure de la couche 304. De manière générale, la couche 114 est déposée de sorte à protéger les plots 306, les billes de soudure 702 et les plots 700 contre l'oxydation.

Un avantage de la boîte de culture 100 tient au fait que chaque source lumineuse 112 est séparée du fond du puits 102 auquel elle est associée par une distance inférieure à 10 µm. On tire ici profit de l'intégration des sources lumineuses 112 dans la boîte de culture 100 pour réduire la distance entre le fond de chaque puits 102 et la ou les diodes 400 associées. Cela permet par exemple de maximiser l'éclairement auquel sont soumises les cellules de la substance 106 présente dans le puits 102, notamment par rapport à une configuration dans laquelle des sources lumineuses de même puissance que les sources 112 seraient placées sous une boîte de culture usuelle.

Un autre avantage de la boîte de culture 100 tient au fait que les diodes électroluminescentes organiques 400 sont à l'origine d'un dégagement de chaleur plus faible que celui que l'on obtiendrait en utilisant par exemple des diodes électroluminescentes inorganiques. Cela permet par exemple de limiter les risques de dégradation de la substance biologique 106 contenue dans les puits 102 sous l'effet de la chaleur dégagée lors de l'émission de lumière par les diodes 400.

Un autre avantage de la boîte de culture 100 tient au fait que des connexions électriques situées en face supérieure du substrat 202 sont reportées en face inférieure du substrat 202 par l'intermédiaire des vias traversants 300. Cela permet en particulier de protéger les circuits 200 contre des risques d'agressions pouvant par exemple résulter de l'utilisation de substances biologiques 106 salines. La présence, dans la boîte 100, de la couche 500 de revêtement dur permet notamment d'éviter que la substance biologique 106 n'entre en contact avec les circuits 200 ou les diodes 400. Cela procure par exemple une protection supérieure par rapport à une configuration dans laquelle la couche 500 serait ouverte pour exposer des éléments de reprise de contact situés du côté de la face supérieure du substrat 202.

Un autre avantage de la boîte de culture 100 tient au fait que son utilisation est susceptible de permettre un gain de temps, par exemple dans le cadre de procédés de séquençage comportant des étapes répétées en boucle, base par base, jusqu'à ce que tout un brin d'ADN (acide désoxyribonucléique) soit séquencé. À titre d'exemple, l'utilisation de la boîte de culture 100 peut permettre un gain de temps dans un cas où des fluorophores, associés à des bases complémentaires venant se greffer sur le brin d'ADN, sont excités chacun par une longueur d'onde différente. Dans ce cas, trois couleurs sont par exemple séquentiellement « lues » pour déterminer la localisation des trois premières bases, une absence d'émission signalant la présence de la quatrième base.

On peut en outre tirer profit de la boîte de culture 100 dans des applications de synthèse d'ADN. À titre d'exemple, l'utilisation de la boîte de culture 100 peut permettre de s'affranchir de procédés complexes et chronophages, tels que des procédés comprenant des étapes de protection et de déprotection par masque microstructuré (par exemple de type résine). L'utilisation de la boîte de culture 100 dans des applications de ce type peut par ailleurs permettre d'écarter des risques d'erreurs liés, par exemple, à une exposition de la substance biologique 106 à de la lumière ultraviolette ou à des solvants agressifs.

Divers modes de réalisation, modes de mise en œuvre et variantes ont été décrits. La personne du métier comprendra que certaines caractéristiques de ces divers modes de réalisation, modes de mise en œuvre et variantes pourraient être combinées, et d'autres variantes apparaîtront à la personne du métier. En particulier, la personne du métier est en mesure d'adapter le mode de mise en œuvre du procédé de fabrication de la boîte 100 décrit ci-dessus à un cas où chaque source lumineuse 112 comporte plusieurs diodes.

Enfin, la mise en œuvre pratique des modes de réalisation, modes de mise en œuvre et variantes décrits est à la portée de la personne du métier à partir des indications fonctionnelles données ci-dessus. En particulier, la réalisation des diodes électroluminescentes organiques 400 est à la portée de la personne du métier à partir des indications ci-dessus.

## Revendications

1. Boîte de culture (100) comportant :
- une matrice de sources lumineuses (112) formée dans et sur un substrat semiconducteur (202) ;
- une couche (500) transparente de planarisation revêtant la matrice de sources lumineuses ; et
- une matrice de puits (102) formée sur ladite couche, chaque source lumineuse étant située à l'aplomb d'un puits.

2. Boîte selon la revendication 1, dans laquelle ladite couche (500) est en un matériau organosilicié photoréticulable.

3. Boîte selon la revendication 1 ou 2, dans laquelle ladite couche (500) présente une épaisseur comprise entre 1 µm et 10 µm.

4. Boîte selon l'une quelconque des revendications 1 à 3, dans laquelle la matrice de puits (102) est en polydiméthylsiloxane.

5. Boîte selon l'une quelconque des revendications 1 à 4, dans laquelle chaque source lumineuse (112) comporte au moins une diode électroluminescente organique (400).

6. Boîte selon la revendication 5, dans laquelle chaque source lumineuse (112) comporte une diode (400) destinée à émettre une lumière blanche.

7. Boîte selon la revendication 5 ou 6, dans laquelle chaque diode (400) est commandable individuellement.

8. Boîte selon la revendication 5 ou 7, dans laquelle chaque source lumineuse (112) comporte trois diodes (400) destinées à émettre respectivement une lumière de couleur rouge, une lumière de couleur verte et une lumière de couleur bleue.

9. Boîte selon l'une quelconque des revendications 1 à 8, dans laquelle la matrice de sources lumineuses (112) est située du côté d'une première face du substrat (202), opposée à une deuxième face du substrat comportant des plots (306) de connexion.

10. Boîte selon la revendication 9, dans laquelle les plots (306) de connexion sont connectés à la première face du substrat (202) par des vias traversants (300).

11. Boîte selon l'une quelconque des revendications 1 à 10, dans laquelle chaque source lumineuse (112) comporte un circuit (200) de commande indépendant des autres sources lumineuses.

12. Boîte selon l'une quelconque des revendications 1 à 11, dans laquelle le fond et/ou les parois latérales d'au moins une partie des puits (102) de la matrice sont fonctionnalisés.

13. Procédé de fabrication d'une boîte (100) selon l'une quelconque des revendications 1 à 12.

14. Procédé selon la revendication 13, comportant les étapes suivantes :
- former la matrice de sources lumineuses (112) dans et sur le substrat semiconducteur (202) ;
- revêtir la matrice de sources lumineuses de la couche (500) transparente de planarisation ; et
- former en surface de ladite couche un puits (102) à l'aplomb de chaque source lumineuse.
